Europäisches Patentamt

(19) European Patent Office

Office européen des brevets

(11) Publication number: **0 058 564**
**B1**

(12) **EUROPEAN PATENT SPECIFICATION**

(45) Date of publication of patent specification: **11.06.86**

(21) Application number: **82300781.0**

(22) Date of filing: **16.02.82**

(51) Int. Cl.⁴: **A 61 N 1/40, A 61 N 1/42**

(54) **Improvements in or relating to electrotherapeutic apparatus.**

(30) Priority: **16.02.81 GB 8104819**

(43) Date of publication of application:
**25.08.82 Bulletin 82/34**

(45) Publication of the grant of the patent:
**11.06.86 Bulletin 86/24**

(84) Designated Contracting States:
**AT BE CH DE FR GB IT LI LU NL SE**

(56) References cited:
**CH-A- 275 614**
**FR-A-1 255 797**
**FR-A-2 207 133**
**FR-A-2 386 912**
**FR-A-2 387 526**
**FR-A-2 391 738**
**FR-A-2 445 151**
**US-A-3 952 751**

(73) Proprietor: **THERAFIELD HOLDINGS LIMITED**
**St. Georges Chambers 1 Athol Street**
**Douglas, Isle of Man (GB)**

(72) Inventor: **Fellus, Victor Marcel**
**19 Rue du Dr. Arnaudet**
**F-92190 Meudon (FR)**

(74) Representative: **Keltie, David Arthur et al**
**Baron & Warren 18 South End Kensington**
**London W8 5BU (GB)**

Courier Press, Leamington Spa, England.

## Description

The present invention relates to electrotherapeutic apparatus.

Such apparatus can be applied in very many fields and in particular in all those where research, development and industry require the application of an electromagnetic field under extremely accurate conditions. Accordingly there are many kinds of apparatus in the prior art for generating electrical currents or for producing magnetic electrical or electromagnetic fields. Such apparatus may operate either as current generators or as field emitters and may do so for either temporary or long-term use on the premises of the person giving treatment or by the patient's bed. For a better understanding of the apparatus with which the present invention is concerned the basic vocabulary will be reviewed taking the example of medical and other biological applications as a basis.

When use is made of electrical currents, at least two electrodes are employed, these electrodes being applied to the patient, who becomes a conductive element in the circuit. This chiefly products various physical and chemical effects (a thermal Joule effect, ionophoresis which provides for the passage of ions through the tissues, etc.) and nerve effects (a motor exciting effect). Amongst the electrical currents used are galvanic (continuous) and faradic and interrupted galvanic (pulsed).

Use has also been made in the prior art, of what have for long been known as "short waves" which are generated by so-called short-wave diathermy apparatus. One way of applying short-waves to a patient is to create a capacitive, and thus purely electrical, field by means of electrodes which may be in the form of plates or flexible pads which are positioned on either side of, and at a distance from, the patient, the patient becoming a dielectric element. The dielectric losses then give rise to heating of the body tissues. Another way of applying "short-waves" is to produce an electric field using only one electrode or plate with the patient forming an earth, spaced from the body which, again, gives rise to heating of the body tissues and could result in a sparking phenomenon between patient and electrode if the apparatus is not used correctly. Further, "short-waves" have been applied by induction coils, which may be formed by coiling a co-axial cable, and which produce a magnetic field that generates eddy currents within the body which are transformed into heat. As with plate and pad electrodes, the induction coils must be spaced from the patient if harmful effects, such as burns are to be avoided. All such "short-wave" treatments are based on the premise that the therapeutic effect is dependent upon the heating action which is ensured by high operating voltages and a continuous emission.

Apparatus is also known for producing pulsed electromagnetic waves which do not produce any significant thermal effects on the patient's body. With such apparatus, the therapeutic effect is considered to be due to the applied electrical forces that are many times higher than those possible with short-wave diathermy apparatus since heat tolerance is no longer a factor. The pulsed electromagnetic waves are applied to a patient's body, by means of a coil-type electrode which must be positioned at a distance from the body, in bursts of high intensity requiring the use of even higher voltages than the short-wave diathermy apparatus to achieve the higher electrical forces considered to be necessary to obtaining the desired therapeutic effect. One form of such apparatus utilises voltages in the range of 600 to 2000 volts, has a peak power of 1400 watts and the pulsed waves produce an average induced power of about 40 watts. The electrode transfers this energy to the patient in the form of an electromagnetic field which is of high energy at the electrode.

It is also known to provide apparatus for emitting high-frequency electromagnetic waves, said apparatus comprising low-voltage power supply means, circuit means for producing high-frequency, electrical signals, said circuit means having an input and an output, means for connecting said input to said power supply means, and at least one antenna which is applicable directly to a patient's body and which is connected or connectible to the output of said circuit means, for producing at the antenna a low-energy high frequency electromagnetic field without significant thermal effects on the body, said antenna comprising at least one conductor which is carried by a support of an electrically insulating material which includes a length of conductive material arranged in a predetermined pattern. Such low-voltage apparatus can utilise voltages of less than 50 volts and energies of less than 100 mW per $cm^2$ of receiving surface, thereby enabling harmful thermal effects to be avoided, bulk to be considerably reduced and high-frequency emitting antenna to be used which can be placed in contact with the patient's body and which do not give to dangers, such as sparking, as can occur with high energy apparatus. It will be appreciated that an antenna of such apparatus does not set up a flow of electrical current between the apparatus and the patient, as is the case with low-frequency current emitting electrodes which operate in direct electrical contact, but produces an electromagnetic field in the body and does not need a cooperating antenna to produce the electromagnetic field.

The document FR—A—2386912 discloses such an electrotherapeutic apparatus for producing and applying to a patient's body a pulsed, low energy, high-frequency electromagnetic field. This apparatus further comprises means for producing pulsed low-frequency electrical currents which can be applied to the patient's body simultaneously or alternatingly with the high-frequency electromagnetic field pulses.

The present invention has for an object to provide electrotherapeutic apparatus capable of treating a patient in two separate modes.

Accordingly the present invention consists in electrotherapeutic apparatus which is capable of applying a high-frequency electrical field to a patient's body, characterized by means for generating and applying to a patient's body a pulsed high frequency electrical field, means for generating and applying to the patient a pulsed magnetic field independent from the high-frequency electrical field, and means for coordinating the pulses of the magnetic field with the pulses of the high-frequency electrical field, whereby the pulses of the magnetic and high-frequency electrical fields can be applied in a predetermined relationship.

Preferably the means for applying the electrical field and the magnetic field comprise a pair of applicator electrodes applicable directly to a patient's body, one applicator electrode being a neutral electrode, and the other applicator electrode comprising at least one magnetic coil for generating a magnetic field and an active electrode for cooperating with the neutral electrode to generate an electrical field.

In order that the present invention may be more readily understood, reference will now be made by way of example to the accompanying drawings, in which:

Figure 1 is a schematic block diagram of electrotherapeutic apparatus constructed in accordance with the present invention,

Figures 2 and 3 are respectively upper and lower perspective views of an electrode for use with the apparatus of Figure 1.

Figure 4 is a schematic section showing two electrodes in position on a patient's body,

Figure 5 is a block diagram of the apparatus shown in Figure 1,

Figure 6 is a circuit diagram showing the components of the block diagram of Figure 5, and

Figure 7 shows some explanatory waveforms.

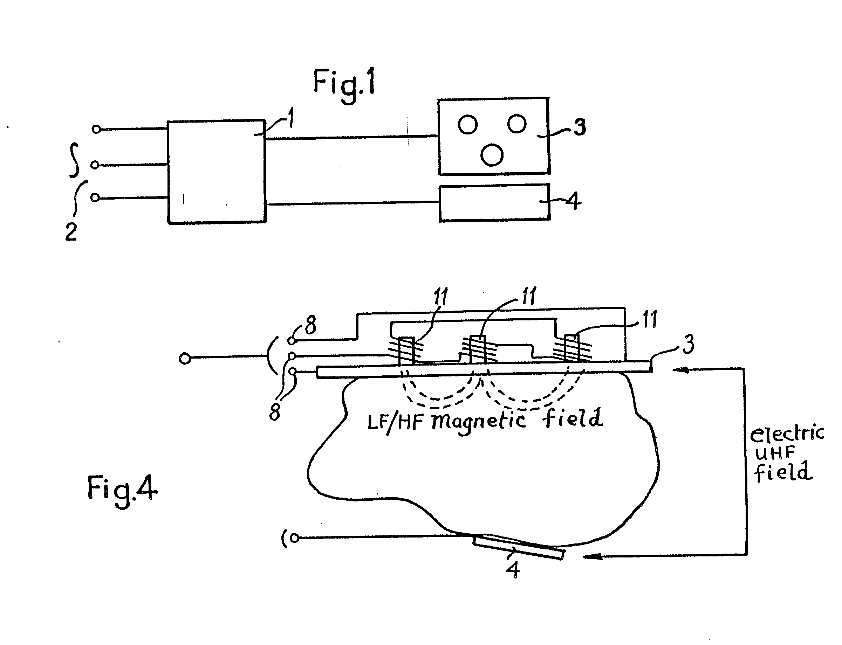

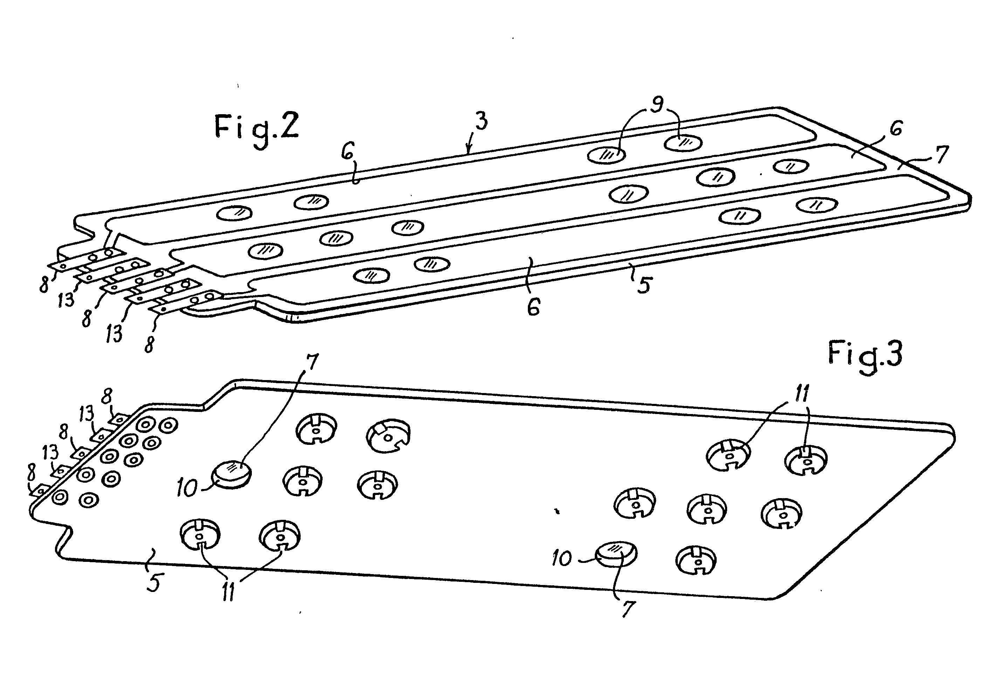

Referring now to the drawings, Figure 1 shows electrotherapeutic apparatus constructed in accordance with the present invention and comprising a generator 1 taking power from a mains supply 2 and supplying energising current to a pair of electrodes 3 and 4. In Figure 1 electrode 3 is an active electrode and is shown in greater detail in Figures 2 and 3, whilst electrode 4 is a neutral electrode.

Referring now to Figures 2 and 3, electrode 3 comprises a substantially rectangular sheet of flexible elastomeric material carrying on the operative face thereof, that is the face which is to be applied to a patient's skin, three individual electrodes 6. Each electrode 6 comprises a metallic strip bonded or adhered to a thin electrically insulating sheet 7 of thermoplastics material which is in turn bonded or adhered to the elastomeric sheet 5. Each strip electrode 6 is connected to an appropriate connector 8 located at one end of the sheet 5. It will be seen that each electrode 6 has a plurality of circular apertures 9 therein which expose the insulating sheet 7. In Figure 3 of the drawings it will also be seen that sheet 5 has a plurality of circular portions 10 removed therefrom so as to provide a series of openings in the sheet 5 which are in register with the circular apertures 9 in the strip electrodes 6. The flexible sheet 7 thus forms a window at each of the openings in sheet 5. A magnetic coil 11 comprising a disc-like ferrite magnetic core is housed in each opening 10 in the sheet 5 and is secured by a suitable adhesive to the sheet 7. One opening 10 is shown without a magnetic core purely for the purposes of illustration. Each magnetic core has a substantially annular recess surrounding a central pole-piece about which is wound a wire coil forming part of the magnetic coil 11 and in turn connectable to connectors 13 located adjacent the connectors 8. The wire coils of the magnetic coils 11 associated with a strip electrode 6 are connected in series. The neutral electrode 4 may be made from a backing sheet similar to sheet 5 of electrode 3 and which carries only a single metallic strip electrode. However it is also possible for the electrode 4 to carry ferrite cores similar to those mounted in the electrode 3. It will be appreciated that in operation of the device the electrodes 3 and 4 will set up high frequency electrical fields therebetween, the generation of such fields requiring the use of two electrodes whilst in the magnetic coils set up oscillating magnetic fields in the patient's body with each magnetic coil operating independently.

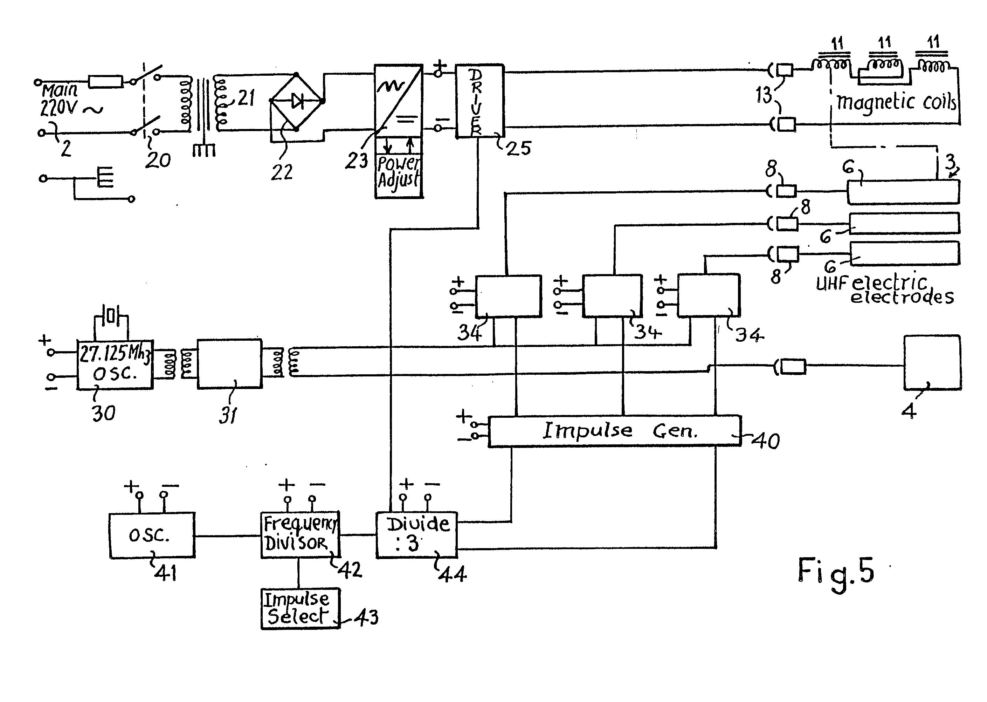

In use the electrodes 3 and 4 are placed on either side of, or at least at spaced positions on, the portion of a patient's body to be treated. This is shown diagrammatically in Figure 4. The generator 1 is desired to supply power to the electrodes 3 and 4 in such a manner that the patient can be treated simultaneously in the two entirely different ways already mentioned. Thus, by means of the strip electrode 6 and the neutral electrode 4 that portion of the patient's body located between the two electrodes 3 and 4 can be subjected to a UHF electric field whilst by means of the magnetic coils 11 that portion of the patient's body adjacent the coils 11 can be treated with either low or high frequency magnetic fields. Thus the generator 1 comprises circuits for supplying appropriate energising currents to both the magnetic coils 11 and the strip electrodes 6 and for controlling the relationship between the application of the two types of treatment.

Referring now to Figure 5 of the accompanying drawings it can be seen that the main supply 2 (shown in Figure 1) is connected via a suitable switch 20 to the input winding of a transformer 21, the output winding of which is connected to two terminals of a diode bridge 22, the outputs of which supply a full-wave rectified output to a power adjust circuit 23. A suitable filter or smoothing circuit may be inserted between the diode bridge and circuit 23. The output from circuit 23 is supplied to a drive circuit 25 which in turn gives an output in the form of a pulsed waveform which is supplied to the coils of the magnetic coils 11 of the active electrode 3. The frequency of the output waveform from the driver circuit 25 is controlled in a manner which will be described hereinafter so as to coordinate application of magnetic energy to the patient with the application of UHF fields. Thus, the circuit for driving the strip electrodes 6 so as to provide a varying HF electrical field between the strip electrodes 6 and the neutral electrode 4 comprises a quartz crystal oscillator 30 adapted to generate a 27.125 MHz. output signal which is supplied to an impedance adaptor 31 and then to the neutral electrode 4 via one line and via another line to three similar matching circuits indicated at 34. Each matching circuit is connected to one of the strip electrodes forming part of the active electrode 3.

The strip electrodes 6 are energised intermittently at intervals determined by an impulse generator 40 in turn controlled by a circuit comprising a variable oscillator 41 providing a train of pulses to a frequency division circuit 42. The output of the frequency division circuit 42 is associated with an impulse select circuit 43 and is connected to a divide-by-three circuit 44 one output of which is taken to the driver circuit 25 for gating the output signals to the coils of the magnetic coils 11 in the active electrode 3. The three outputs of the divide-by-three circuit 44 are also supplied to the impuse generator 40 to control the latter.

It will thus be seen that in operation of the device a patient will be subjected to alternate bursts of magnetic energy from coils 11 and their associated cores and high frequency electric fields generated between strip electrodes 6 and neutral electrode 4.

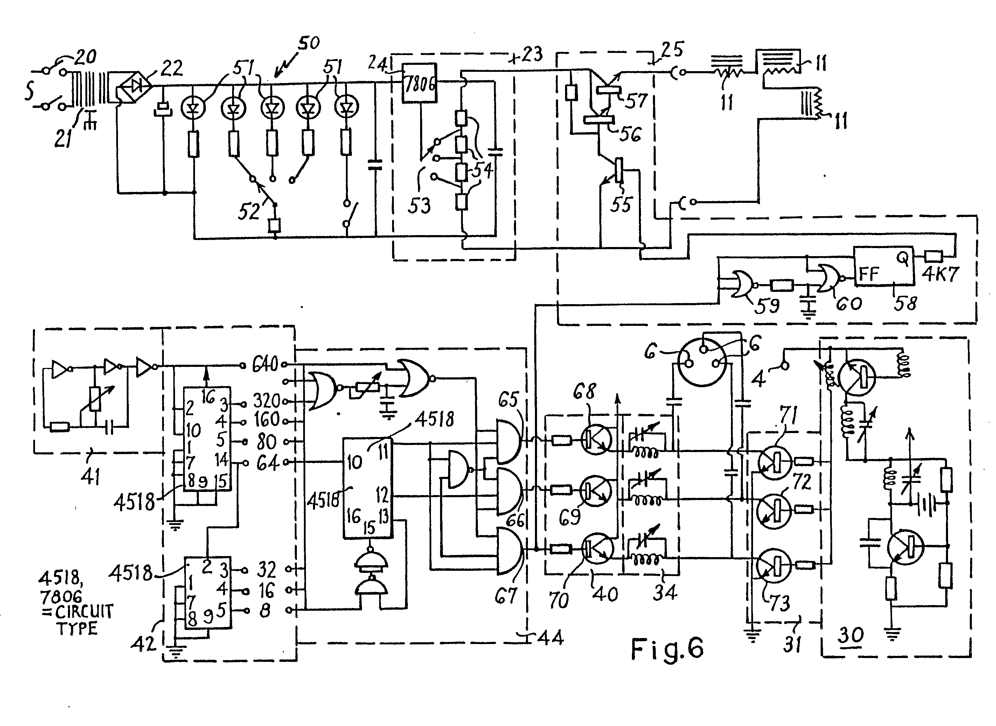

Referring now to Figure 6 this shows the circuit components of the block diagram of Figure 5. Thus, in this figure integers which are common to those of Figure 5 have been given the same reference numerals. The output of diode bridge 22 is connected to the power adjust circuit 23 via a display circuit generally indicated at 50 and including five parallel photodiodes 51; the outputs of the three central ones of the photodiodes 51 are connected to contacts of a three-position switch 52 which is ganged to a switch 53 in the power adjust circuit 23. The power adjust circuit 23 includes a voltage regulator 24 one output of which is selectably connectable by switch 53 to different points in a resistor chain of four resistors 54 connected in series so that the output power of the circuit can be set at three different levels by switch 53. When the apparatus is in use adjustment of the main switch 53 will correspondingly adjust the switch 52 so that a visible indication will be given of the power selected by the power adjust circuit 23 by the photodiode connected into circuit by switch 52 lighting up. Naturally there are many alternative methods of giving a visible indication.

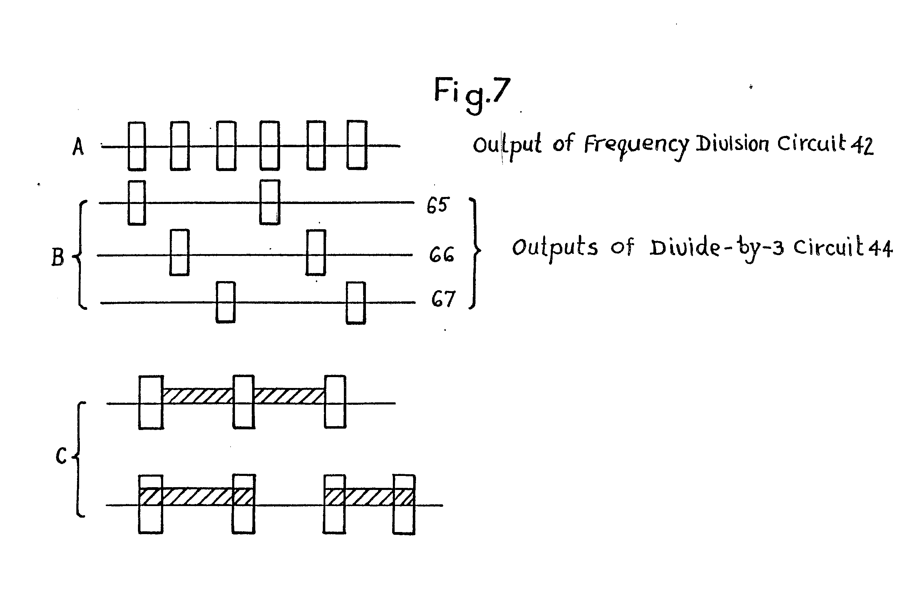

The output of the power adjust circuit 23 is taken to the driver circuit 25 which comprises three transistors 55, 56, 57. The transistor 55 is a control transistor the base of which is connected to the Q-output of a flip-flop 58 whilst the transistors 56 and 57 are connected in cascade. One input of flip-flop 58 is connected directly to one output of the divide-by-three circuit 44 whilst the other input is connected to a pair of logic gates 59 and 60 connected in series. Logic gate 59 has its two inputs connected to the same output of divide-by-three circuit 44 as the other input of flip-flop 58. The variable oscillator 41 supplies a train of pulses of selected frequency to the input of frequency division circuit 42. As shown this circuit is capable of giving a range of output frequencies. In the present embodiment these are 640, 320, 160, 80, 64, 32, 16 and 8. The divide-by-three circuit 44 takes the train of pulses from. the frequency division circuit 42 and it distributes these sequentially to three output points 65, 66 and 67 under the control of the I.C. circuit type 4518 shown at 46 which basically provides the impulse select circuit 43. This can be seen from Figure 7 where waveform A shows the output of circuit 42, and B the outputs from the output points 65, 66 and 67. The three signals from the outputs 65, 66 and 67 are respectively applied to the bases of transistors 68, 69 and 70 in impulse generator 40 which in turn feed the matching circuit 34 for application to the strip electrodes 6. The signals from the divide-by-three circuit and the impulse generator 40 are intended to control the application to the strip electrodes 6 of the high fequency signals generated by the oscillator 30. As can be seen from Figures 5 and 6 an output of oscillator 30 is taken to the neutral electrode 4, and as can be seen from Fig. 6 an output is also connected to the bases of three power transistors 71, 72 and 73 for application to the electrode 6 under the control of the signals from the impulse generator 40.

The relationship between the two forms of energy which can be applied to the patient can best be understood by reference to Figure 7c. This shows two separate ways in which the phase and frequency of the high-frequency electrical signals which are supplied to strip electrodes 6 can be related to the phase and frequency of the current for driving the wire coils of the coils 11 to generate the magnetic field. In the two waveforms shown the current to the wire coils of the magnetic coils 11 is represented by hatching. Thus, in the first of the two waveforms there is applied to the patient magnetic energy and UHF fields in bursts which are of the same frequency but with no overlap between the two modes of treatment although the patient is continuously treated with one or the other forms of energy. In the second waveform it will be seen that the frequency of application of the magnetic field via the magnetic coils 11 is half of that of the application of the electrical field by the strip electrodes 6 and neutral electrode 4. It will of course be fully appreciated that by suitably selecting the logic circuitry interconnecting the output of the divide-by-three circuit 44 and the driver circuit 25 that the relationship between the application of the magnetic energy and of the UHF fields can be varied as desired. However in the present embodiment it will be realised that there is always some fixed relationship as the two forms of energy are controlled by control signals derived from a single oscillator. Furthermore it is possible to alter the intensity of the magnetic field independently of the other mode of treatment.

The present invention, applicable to all forms of electrotherapeutic apparatus, is particularly intended for use with low voltage apparatus utilising voltages of less than 50 volts and energies of less than 100 mw per cm² of receiving surface, thereby enabling harmful thermal effects to be avoided, bulk to be considerably reduced with regard to high voltage apparatus and antenna or applicator electrodes to be used which can be placed in contact with the patient's body.

When the apparatus described herein is in use a patient will not feel the magnetic or electrical fields generated by the apparatus. However the magnetic coils 11 will in operation produce a degree of vibration which can be felt by the patient and will provide confirmation to him that he is in fact being treated.

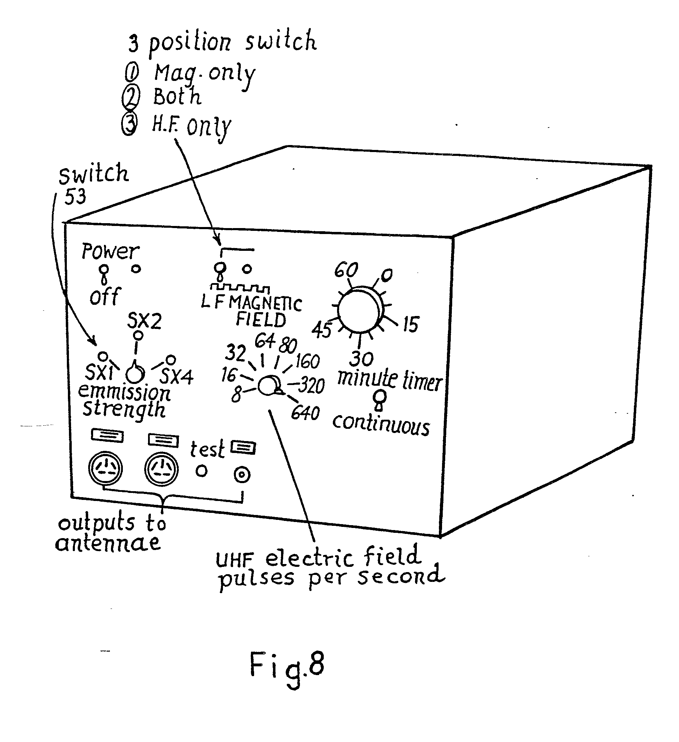

Figure 8 is a perspective view of the apparatus just described and shows a substantially rectangular casing.

Referring now to Figure 8 of the accompanying drawings, this shows the outward appearance of a practical embodiment of the electrotherapeutic apparatus which has been described with reference to Figures 1 to 7.

**Claims**

1. Electrotherapeutic apparatus which is capable of applying a high-frequency electrical field to a patient's body, characterized by means (30, 31 and 3) for generating and applying to a patient's body a pulsed high frequency electrical field, means (22, 23, 25 and 11) for generating and applying to the patient a pulsed magnetic field independent from the high-frequency electrical field, and means (40, 41, 42 and 43) for coordinating the pulses of the magnetic field with the pulses of the high-frequency electrical field, whereby the pulses of the magnetic and high-frequency electrical fields can be applied in a predetermined relationship.

2. Apparatus as claimed in claim 1, characterized in that said predetermined relationship involves either the pulses of the magnetic field being disposed between pulses of the high-frequency electrical field or the pulses of the magnetic field being in overlapping relationship with pulses of the high-frequency electrical field.

3. Electrotherapeutic apparatus as claimed in claim 1 or 2, and characterized in that there is provided a first circuit means (30, 31) for generating high-frequency electrical signals, applicator means (3) for applying said signals to a patient's body to produce therein the pulsed high-frequency electrical field, a second circuit means (22, 23, 25 and 11) for generating and for applying to a patient's body the pulsed magnetic field which is independent from said high-frequency electrical field, said second circuit means including a first circuit (22, 23) for generating electrical output signals, a drive circuit (25) for gating said electrical output signals from said first circuit to produce an output wave form and another circuit (11) which is energized by said output wave form to produce said pulsed magnetic field, and a third circuit means (40, 41, 42 and 43) connected to said first circuit means (30, 31 and 3) and to said drive circuit (25) to coordinate the output wave form from said drive circuit with an output waveform of said high-frequency electrical signals from said first circuit means.

4. Apparatus as claimed in claim 3, characterized in that said third circuit means (40, 41, 42 and 43) for coordinating the output wave forms from the drive circuit (25) and said first circuit means (30, 31 and 6) comprises an impulse generator (40) controlled by the output of a frequency divider circuit (42) one input of which is connected to the output of a variable oscillator (41).

5. Apparatus as claimed in claim 4, characterized in that said third circuit means (40, 41, 42 and 43) further comprises an impulse select circuit (43) for controlling the operation of the frequency divider circuit (42).

6. Apparatus as claimed in claim 4 or 5, characterized in that said third circuit means further comprises a divider circuit (44) having an input connected to the frequency divider circuit (42), having one output connected to the driver circuit (25) and having other outputs connected to the impulse generator (40).

7. Apparatus as claimed in any one of claims 4 to 6, characterized in that said first circuit means (30, 31 and 3) includes an H.F. oscillator (30) and at least one matching circuit (34) which is connected to said oscillator (30) and whose operation is controlled by said impulse generator (40).

8. Apparatus as claimed in any one of claims 3 to 7, characterized in that said applicator means (3) comprises at least one active electrode (6) which is applicable directly to a patient's body and which includes said another circuit (11) of said second circuit means (22, 23, 25 and 11), said another circuit comprising at least one magnetic coil (11), and in that a neutral electrode (4) which co-operates with the active electrode (3) in the production of the high-frequency electrical field is applicable directly to a patient's body.

9. Apparatus as claimed in claim 8, characterized in that said active electrode comprises a flexible sheet (5, 7), at least one conductive strip (6) mounted on the sheet (5, 7) which said at least one magnetic coil (11) being mounted in a recess (9) in said sheet (5, 7) and comprising a magnetic core about which is wound a wire coil.

10. Apparatus as claimed in claim 8 or 9, characterized in that the neutral electrode (4) comprises at least one strip electrode mounted on or incorporated in another flexible sheet.

**Revendications**

1. Dispositif d'électrothérapie destiné à appliquer sur le corps d'un patient un champs électrique de haute fréquence, caractérisé en ce qu'il comporte des moyens (30, 31 et 3) pour générer et pour appliquer sur le corps d'un patient un champs électrique pulsé de haute fréquence, des moyens (22, 23, 25 et 11) pour générer et appliquer sur le corps du patient un champs magnétique pulsé indépendant du champs électrique de haute fréquence, et des moyens (40, 41, 42 et 43) pour coordonner les impulsions du champs magnétique et les impulsions du champs électrique de haute fréquence de sorte que les impulsions des champs magnétique et électrique de haute fréquence puissent être appliquées suivant une relation prédéterminée.

2. Dispositif suivant la revendication 1, caractérisé en ce que ladite relation prédéterminée consiste soit à appliquer les impulsions du champs magnétique entre les impulsions du champs électrique de haute fréquence soit à appliquer les impulsions magnétiques en superposition avec les impulsions du champs électrique de haute fréquence.

3. Dispositif d'électrothérapie suivant l'une quelconque des revendications 1 ou 2, caractérisé en ce qu'il comporte un premier circuit (30, 31) pour produire des signaux électriques de haute fréquence, des moyens d'application (3) pour appliquer ces signaux sur la corps du patient pour produire un champs électrique pulsé de haute fréquence, un deuxième circuit (22, 23, 25 et 11) pour produire et appliquer sur le corps du patient un champs magnétique pulsé qui est indépendant dudit champs électrique de haute fréquence, ledit deuxième circuit (22, 23) produisant des signaux électriques de sortie, un circuit pulsatoire (25) pour moduler lesdits signaux électriques de sortie pour produire une pulsation de sortie et un autre circuit (11) excité par la pulsion de sortie pour produire ledit champs magnétique pulsé, et un troisième circuit (40, 41, 42 et 43) relié audit premier circuit (30, 31 et 3) et audit circuit pulsatoire (25) pour coordonner l'impulsion produite par ledit circuit pulsatoire avec une impulsion des signaux électriques de haute fréquence produits par ledit premier circuit.

4. Dispositif suivant la revendication 3, caractérisé en ce que ledit troisième circuit (40, 41, 42 et 43) destiné à coordonner les impulsions de sortie du circuit pulsatoire (25) et dudit premier circuit (30, 31 et 6), comporte un générateur d'impulsions (40) commandé par les signaux de sortie d'un circuit diviseur de fréquence (42) dont l'une des entrées est reliée à la sortie d'un oscillateur ajustable (41).

5. Dispositif suivant la revendication 4, caractérisé en ce que ledit troisième circuit (40, 41, 42 et 43) comporte de plus un circuit de sélection d'impulsions (43) pour contrôler le fonctionnement du circuit diviseur de fréquence (42).

6. Dispositif suivant l'une quelconque des revendications 4 ou 5, caractérisé en ce que ledit troisième circuit comporte de plus un circuit diviseur (44) dont l'une des entrées est reliée au circuit diviseur de fréquence (42) et qui présente une sortie reliée au circuit diviseur (25) et des sorties reliées au générateur d'impulsions (40).

7. Dispositif suivant l'une quelconque des revendications 4 à 6, caractérisé en ce que ledit premier circuit (30, 31 et 3) comporte un oscillateur HF (30) et au moins un circuit de commutation (34) relié audit oscillateur (30) et dont le fontionnement est contrôlé par ledit générateur d'impulsions (40).

8. Dispositif suivant l'une quelconque des revendications 3 à 7, caractérisé en ce que lesdits moyens d'application (3) comprennent au moins une électrode active (6) qui peut etre appliquée directement sur le corps du patient et qui comporte ledit autre circuit (11) dudit deuxième circuit (22, 23, 25 et 11), ledit autre circuit comprenant au moins une bobine magnétique (11), étant prévue une électrode neutre (4) coopérant avec ladite électrode active (3) pour la production du champs électrique de haute fréquence et qui peut être appliquée directement sur le corps du patient.

9. Dispositif suivant la revendication 8, caractérisé en ce que ladite électrode active comporte une feuille souple (5, 7), au moins une bande conductrice (6) montée sur cette feuille (5, 7), au moins l'une des bobines magnétiques (11) étant montée dans un évidement (9) prévu dans ladite bande (5, 7) et comprenant un noyau magnétique autour duquel est disposé un enroulement.

10. Dispositif suivant l'une quelconque des revendications 8 ou 9, caractérisé en ce que l'électrode neutre (4) comporte au moins une électrode en forme de bande montée sur une autre feuille souple ou incorporée dans celle-ci.

**Patentansprüche**

1. Elektrotherapeutisches Gerät, durch das ein hochfrequentes elektrisches Feld auf einen Patientenkörper übertragen werden kann, gekennzeichnet durch Mittel (30, 31 und 3) zur Erzeugung und zur Übertragung eines pulsierenden, hochfrequenten, elektrischen Feldes auf einen Patientenkörper, durch Mittel (22, 23, 25 und 11) zur Erzeugung und Übertragung eines vom hochfrequenten, elektrischen Feld unabhängigen, pulsierenden Magnetfeldes auf den Patienten, und durch Mittel (40, 41, 42 und 43) zur Gleichschaltung der Magnetfeldimpulse und der Impulse des hochfrequenten elektrischen Feldes, wodurch die Impulse des magnetischen und des hochfrequenten elektrischen Feldes in einem festgelegten Verhältnis übertragen werden können.

2. Gerät nach Anspruch 1, dadurch gekennzeichnet, daß das festgelegte Verhältnis entweder die Magnetfeld-impulse zwischen den Impulsen des hochfrequenten elektrischen Feldes enthält oder die Magnetfeldimpulse befinden sich in Überlappung mit den Impulsen des hochfrequenten elektrischen Feldes.

3. Elektrotherapeutisches Gerät nach Anspruch 1 oder 2, dadurch gekennzeichnet, daß eine erste Schaltung (30, 31) zur Erzeugung hochfrequenter elektrischer Signale vorgesehen ist, daß Mittel (3) zur Anwendung der Signale auf einen Patientenkörper vorgesehen sind, um in ihm das pulsierende hochfrequente elektrische Feld zu erzeugen, daß eine zweite Schaltung (22, 23, 25, 11) zur Erzeugung und Anwendung des pulsierenden, von dem hochfrequenten elektrischen Feld unabhängigen magnetischen Feldes auf den Patientenkörper vorgesehen ist, wobei die zweite Schaltung einen ersten Stromkreis (22, 23) zur Erzeugung elektrischer Ausgangssignale enthält, daß ein Antriebsstromkreis (25) zur Steuerung der elektrischen Ausgangssignale aus dem ersten Schaltkreis zur Erzeugung einer Ausgangswellenform und ein weiterer Schaltkreis (11) vorgesehen ist, der durch die Ausgangswellenform erregt wird, um das pulsierende magnetische Feld zu erzeugen, und daß eine dritte Schaltung (40, 41, 42, 43) vorgesehen ist, die an den ersten Schaltkreis (30, 31, 3) und an den Antriebskreis (25) angeschlossen ist, um die Ausgangswellenform aus der Antriebschaltung mit einer Ausgangswellenform der hochfrequenten, elektrischen Signale aus der ersten Schaltung gleichzuschalten.

4. Gerät nach Anspruch 3, dadurch gekennzeichnet, daß der dritte Schaltkreis zur Gleichschaltung der Ausgangswellenform aus dem Antriebsschaltkreis (25) und dem ersten Schaltkreis (30, 31, 6) aus einem Impulsgenerator (40) besteht, der durch den Ausgang einer Frequenz-Teilerschaltung (42) gesteuert wird, dessen einer Eingang mit dem Ausgang eines variablen Oszillators (41) verbunden ist.

5. Gerät nach Anspruch 4, dadurch gekennzeichnet, daß der dritte Schaltkreis (40, 41, 42, 43) weiter einen Impulswählkreis (43) zur Steuerung des Betriebes der Frequenz-Teilerschaltung (42) enthält.

6. Gerät nach Anspruch 4 oder 5, dadurch gekennzeichnet, daß die dritte Schaltung weiter einen Teilerkreis (44) enthält, der einen Ausgang hat, der mit dem Frequenzteiler (42) verbunden ist, welcher einen Ausgang aufweist, der an den Antriebskreis (25) angeschlossen ist und einen zweiten Ausgang aufweist, der an den Impulsgenerator (40) angeschlossen ist.

7. Gerät nach einem der Ansprüche 4 bis 6, dadurch gekennzeichnet, daß die erste Schaltung (30, 31, 3) einen HF-Oszillator (30) und mindestens einen Abstimmkreis (34) enthält, der mit dem Oszillator (30) verbunden ist und dessen Betrieb durch den Impulsgenerator (40) gesteuert wird.

8. Gerät nach einem der Ansprüche 3 bis 7, dadurch gekennzeichnet, daß die Übertragungsmittel (3) aus mindestens einer aktiven Elektrode (6) bestehen, die direkt an den Patientenkörper angelegt wird, und die den zweiten Kreis (11) der zweiten Schaltung (22, 23, 25, 11) enthält, wobei die zweite Schaltung aus mindestens einer Magnetspule (11) besteht und daß eine neutrale Elektrode (4), die mit der aktiven Elektrode (3) bei der Erzeugung des hochfrequenten elektrischen Feldes zusammenarbeitet, direkt an den Patientenkörper angelegt wird.

9. Gerät nach Anspruch 8, dadurch gekennzeichnet, daß die aktive Elektrode aus einer flexiblen Folie (5, 7), mindestens einem leitenden Streifen (6) auf der Folie besteht, mit der mindestens eine Magnetspule (11) in einer Ausnehmung (9) der Folie (5, 7) montiert ist und aus einem Magnetkern besteht, um den eine Drahtspule gewickelt ist.

10. Gerät nach Anspruch 8 oder 9, dadurch gekennzeichnet, daß die neutrale Elektrode (4) aus mindestens einem Elektrodenstreifen besteht, der auf oder in einer weiteren flexiblen Folie montiert ist.

# Fig.1

# Fig.4

LF/HF magnetic field

electric UHF field

0 058 564

Fig. 2

3

9

6

6

7

8

13

8

13

8

5

6

Fig. 3

7

11

10

8

13

8

13

8

11

5

11

10

7

0 058 564

Fig.5

0 058 564

Fig.6

0 058 564

# Fig.7

Output of Frequency Division Circuit 42

Outputs of Divide-by-3 Circuit 44

3 position switch
① Mag. only
② Both
③ H.F. only

Switch
53

Power
o o
off

SX2

SX1 SX4
emmission
strength

LF MAGNETIC
FIELD

32 64 80
160
16
8 320
640

60 0

45 15

30
minute timer
o
continuous

outputs to
antennae

test

UHF electric field
pulses per second

Fig.8

6